# EUROPEAN PATENT SPECIFICATION

(11) **EP 3 538 186 B1**
(45) Date of publication and mention of the grant of the patent: **30.09.2020**
(21) Application number: 16795019.5
(22) Date of filing: 10.11.2016
(51) Int. Cl.: A61M 5/48, A61M 5/168, A61M 5/178

(54) **NEEDLE FOR A SYRINGE, SYRINGE AND CORRESPONDING CONTROL SYSTEM**
NADEL FÜR EINE SPRITZE, SPRITZE UND ZUGEHÖRIGES STEUERUNGSSYSTEM
AIGUILLE DESTINÉE À UNE SERINGUE, SERINGUE ET SYSTÈME DE COMMANDE CORRESPONDANT

(43) Date of publication of application: 18.09.2019
(73) Proprietor: Lightsens Medical SA, 6500 Bellinzona (CH)
(72) Inventor: LEONARDI, Matteo, 6500 Bellinzona (CH); SAPORITO, Andrea, 6702 Claro (CH); QUADRI, Christian, 6512 Giubiasco (CH)
(74) Representative: M. Zardi & Co S.A.
(86) International application number: PCT/EP2016/077343
(87) International publication number: WO 2018/086699

(56) References cited:
- WO-A1-2007/115402
- WO-A1-2012/040543
- WO-A1-2014/085806
- WO-A1-2014/158716
- WO-A2-2007/089948
- JP-A- 2013 009 919
- US-A- 4 411 657
- US-A1- 2008 154 188
- US-A1- 2014 012 226
- US-B1- 9 399 101

## Description

### Field of application

The present invention relates to a needle for a syringe.

The present invention also relates to a syringe, and more particularly to a syringe and to a control system adapted to alert a user if a liquid is injected through the syringe in or near body tissues or body fluids not adapted to be addressed by the liquid.

### Prior art

In daily medical practice, injections of a liquid into a tissue, organ, or body cavity is extremely common, including steroids, local anaesthetics, hyaluronic acid, etc. Examples of injection devices can be found in JP 2013 009919, US 2008/154188, WO 2007/089948, US 2014/012226, WO 2014/158716, US 9,399,101, WO 2014/085806, WO 2007/115402, WO 2012/040543 and US 4,411,657.

In performing injections, it is important to identify the proper location at which injecting the fluid. In the vast majority of cases these injections are performed with a blind technique, i.e. according to external anatomic markers, or under ultrasound, fluoroscopic or CT guidance. However in many cases the definition provided by these imaging techniques is not sufficient to confirm beyond any doubt the exact position of the needle tip.

Even for an experienced operator, an injection carries the intrinsic risk of a needle tip wrong positioning, with consequent injection of a drug in the wrong tissue, organ, or cavity. An important information which can help the operator detect an error in the needle position is the pressure during the injection. The operator try to sense the nature of the tissue he is injecting into by a subjective interpretation of the resistance he feels while pressing on the plunger of the syringe. For example, if the tip of the injecting needle has inadvertently been placed in a tissue or organ with a scarce elastance (such as a tendon or a nerve), during the subsequent injection of the drug the operator will typically encounter a high resistance. On the contrary, if the needle tip is located in a blood vessel, the fluid will flow easily, with a very limited resistance. This is of course not accurate, as based only on a subjective feeling and the experience of the operator and not on objective and measured data.

While in some situation, the wrong site injection consequence will only be a diminished therapeutic effect, in other cases it can have serious consequences. That is for example the case of injecting a local anaesthetic directly in a nerve during a regional anesthesia procedure.

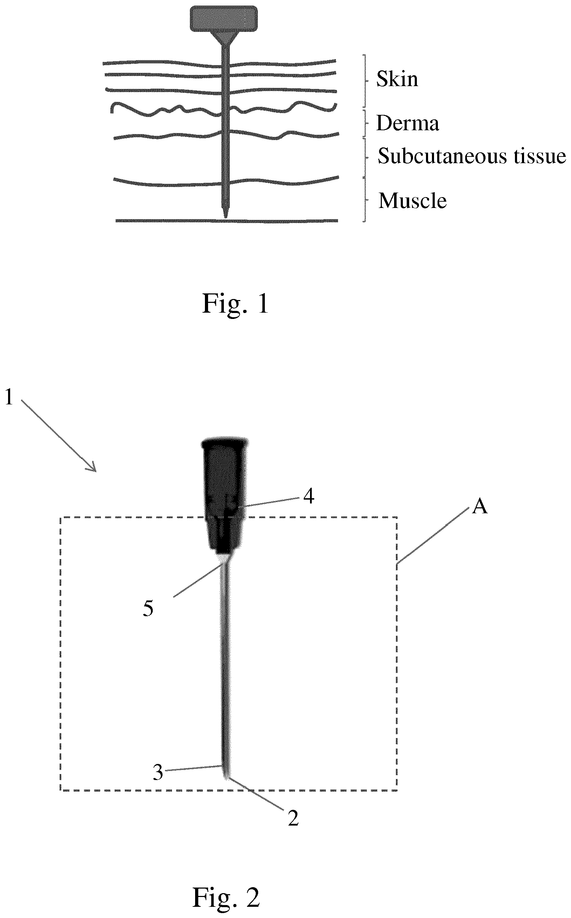

Fig. 1 schematically represents a needle passing through the skin, the derma, subcutaneous tissue and muscle. Such a location, may be the right or wrong location in which injecting the liquid depending on the kind of application. In surgical operations, an anesthetic nerve block may be used to anesthetize the nerves innervating the area of the surgical wound.

Before injecting the local anesthetic, the anaesthesiologist inserts the needle in proximity of the nerves to be blocked. To do so, the anaesthesiologist may insert the needle "blindly", just relying on anatomical landmarks, or he may try to elicit paraesthesia, when the needle touches the nerve; according to another method, the anesthesiologist may use a nerve stimulator to elicit the motor response (twitch) of the nerve. Neurostimulation has largely replaced paresthesia as the primary means of nerve localization in the 1980s. In the last few years the use of ultrasound to visualize the needle and nerves in real time during nerve block procedures has become very popular, and is nowadays widely diffused.

Through any one of the above methods, the anaesthesiologist tries to insert the needle as close as possible to the nerve (since vicinity is necessary for a reliable and effective nerve block). The intrinsic risk is of course to inadvertently puncture the nerve however nowadays cannot be completely excluded by any of the guidance techniques available.

The inadvertent needle tip placement inside a nerve causes two possible damage mechanisms to the neural tissue: the first is direct nerve fibres trauma by the needle; the second is the damage caused by the subsequent injection of the local anesthetic inside the nerve, instead of around the nerve.

The latter situation, called intraneural injection, is thought to be the main cause of the permanent severe nerve damage, which consequent loss of function, which represents the most feared complication of peripheral nerves blocks.

Also in case of central regional anesthesia procedures, such as spinal and epidural anesthesia, which can be performed under real time ultrasound guidance, but are nowadays still performed with anatomical landmarks, a significant cause of iatrogenic morbidity is due to inadvertent injection of the local anesthetic or the drug used (corticosteroids, ialuronic acid, contrast, etc.) in the wrong place (inside a root, either subdural instead of spinal, or vice versa when not completely out of target in the soft tissues). The same can be said with regard to pain therapy procedures, such as epiduroscopies, discographies, indwelling spinal catheters placement, etc.

Finally, another relatively common error, which can lead to dramatic consequences is the inadvertent intravascular injection of local anesthetics or drugs which are not meant for intravenous use. This can cause acute neurotoxicity and cardiac arrest (in case of a local anesthetic) or thromboembolism (in case of certain corticosteroids).

In all these circumstances, a diverse injection pressure can be felt during the injection of the drug, but again this is just a subjective feeling which is not objectivable and measurable.

Sustained and permanent nerve damage represents of course the main complication in regional anesthesia. Although quite rare (about 4:10'000 cases) they are associated to a devastating impact on patients' quality of life, leading to loss of function, anesthesia, dysesthesia, paresthesia and chronic pain syndromes. Moreover even minor nerve lesions associated to temporary side effects are a major cause of claims and are thus one of the main concerns for professionals and hospital administrations with regard to the implementation of regional anesthesia in clinical practice.

When adopted in everyday clinical practice, operators' main concern is thus to manage the two main risks associated to regional anesthesia: unintentional intraneural and intravascular local anesthetic injection. To decrease this risk anesthesiologist and nurse anethesiologist are usually instructed to inject the local anesthetic very slowly and in fractionated boluses after repeated aspiration tests, in order to detects the high injection pressures associated to intraneural injection and the blood aspiration associated to intravascular needle tip positioning. When high pressures are felt on the syringe during injection, the needle tip is assumed to be probably intraneural, the injection is thus immediately interrupted and the needle tip repositioned. Of notice, the increase in injection pressure to levels that can be felt by the operator on the syringe is a late and subjective sign of intraneural injection.

Not all the operators have the same experience and the same perception of what is supposed to be a "normal" or an "abnormal" injection pressure. Moreover, not all the operators reacts in the same way to a supposedly excessive perceived injection pressure, being their judgments affected, among other factors, by their subjective interpretation, the lack of an objective measurement method, the injection method they use, the type of syringe and needle used, the speed of injection. For example, smaller syringes (2-5 mls volume) and smaller calibre needles generate higher injection pressures than bigger syringes and lower gauged needles.

One objective and reproducible method of monitoring and controlling the pressure during nerve blocks is nowadays available, with an injection pressure monitoring device actually present in the market. This method consists in a device featuring a sensor placed on the line between the syringe and the needle. The sensor is hosted inside a case connected to the syringe and the needle via the injection line. The case include a mechanical visual indicator, which should be constantly checked by the operator during the injection in order to detect high injection pressures. The indicator does not show the exact injection pressure generated by the operator, but can just signal three different situations: low injection pressures (visually signalled with a white colour, associated to injection pressures lower than 15 psi, a yellow colour, which signal an initial situation of potential risk, associated to injection pressure between 15 and 20 psi and a red colour, associated to injection pressures superior to 20 psi).

This device allows a certain degree of control on the injection pressure, however presents many problems which prevent it to be a definitive solution to the issues indicated above.

First it poses some ergonomic problems: many operators are disturbed by the presence of the tubes connecting the syringe and the needle to the device, as well as by the presence of the device itself, which alter the usual structure of the leaner system syringe-injection line-needle. This is not a secondary issue, since ergonomy and simplicity are extremely important to the operator, in order to minimize potential errors (namely disconnection between multiple joints leading to leaks along the line altering the accuracy of both the volume injected and the injection pressure) and to have a comfortable and handy system easy to operate. In order to check the pressure he or she is generating along the injection line at any time, the operator must constantly check the device, thus stopping to look the needle insertion point and the ultrasound machine monitor, with consequent risk of unintentional displacement of the needle tip during the injection procedure and following risks of both complications and block failure. For a safe real-time ultrasound guided regional anesthesia procedure ergonomy is actually very important, one its main pillars being to constantly keep in the same visual field the needle and the monitor, checking the screen continuously to early detect local anesthetic spread pattern suggesting unappropriate needle tip positioning. For this reasons, many anesthesiologists nowadays have not adopted the device in their daily clinical practice and they are unlikely to adopt it in the future.

Moreover, this device is also affected by many important limitations with regard to its accuracy, which definitely limit the actual clinical utility of the information provided about injection pressure. As mentioned above, the injection pressure detected by a sensor placed far from the needle tip, along the injection line, is influenced by many factors, including the speed of injection, the size of the needle, the size of the tubes, the size of the syringe, the size and shapes of the device itself, the resistance offered by the needle to the pressure exerted on the plunger by the operator. Due to all this factors, the pressure detected by the sensor of the known device cannot be used to make precise assumption on whether the liquid is injected in the proper location or not. In other words, this known device may give an information or feed-back to the operator on a possible wrong location being instead the location appropriate (false positive) or, on the opposite (and more dangerously), it may give a false information on a possible correct location for the injection when, instead, the location is wrong (false negative). These accuracy issues translate into a lack of sensitivity and specificity in detecting intraneural injections, leading to a potentially significant failure in lowering the incidence of nerve injury.

Furthermore, the indicator gives the information with a certain delay and with low precision. In fact it provides only information about three thresholds, which have been associated to different risk profiles.

The actual real time pressure at the needle tip (which is the information really valuable to the operator) is by any means measured nor shown.

Finally the existing device does not provide any information about an injection pressure lower than expected, which can be associated to important complications of regional anesthesia, namely intravascular or unintentional spinal injection of local anesthetic.

It also does not give the possibility of record the injection pressures generated during the procedures and thus does not constitute a proof of good clinical practice in case of litigation.

Of notice, the existing device is marketed just for regional anestesia peripheral nerve blocks, while injection pressure monitoring constitutes a valuable information in many other medical fields, such as other regional anesthesia procedures (ex. neuraxial blocks), interventional pain therapy procedures, interventional radiology procedures, bioptic procedures, etc.

The problem at the base of the present invention is to provide a needle, a syringe and a system able to provide an accurate, objective, easily understandable, real-time information about a wrong needle tip position, increasing the safety of regional anesthesia, pain therapy and interventional radiology practices, by lowering the incidence of complications due to the injection of the drugs in inappropriate sites, as well as giving to the clinicians the possibility to prove their adherence to good clinical practice recommendations, for instance in case of litigation.

### Summary of the invention

The idea at the base of the present invention is to provide a needle including a pressure sensor within the needle shaft, and measuring in real-time the pressure of the liquid in the shaft during injection. The pressure of the liquid in the shaft depends on the resistance of the body tissue, fluid and elastance of the cavity, tissue or organ in which the liquid is injected.

Advantageously, the pressure sensor detects the pressure close to the body portion in which the needle tip is inserted and therefore with high precision, independently from the pressure drop or biases before and in the needle, being this information really useful to understand whether the injection may be dangerous or not and therefore to decide if continuing or promptly stopping injection to reposition the needle. Depending on the kind of application, a pressure threshold is set and an alarm is emitted if the pressure detected by the sensor during injection is below or above the threshold.

Advantageously, the alarm is given without delay because the pressure sensor detects the pressure immediately close to the structure where the needle is positioned, for instance at the needle tip portion and, when an inappropriate pressure is detected (for instance a pressure above the threshold), the alarm is immediately operated. Advantageously, the alarm is controlled and/or activated electrically without substantial delay with respect to the time in which the pressure is detected.

Moreover, according to an aspect of the present invention, a continuous injection pressure measurement during an injection, allows to draw an injection pressure wave profile for the injection. The injection pressure wave profile may be stored in association with data of the injection, for instance time and date of the injection, and/or reference to a tissue, cavity or organ in which the injection has been done and/or identification information of the doctor who made the injection.

A plurality of injection pressure wave profiles may be collected for each tissue, cavity or organ and used to build or to select an injection pressure wave pattern for each of said tissue, cavity or organ. For instance, among the plurality of injection pressure wave profiles, one or more profiles, corresponding to injection(s) having no negative effects on patients, may be identified as a typical or normal patterns, while one or more patterns, corresponding to an injection causing pains or some other adverse effects on the patient, may be identified as abnormal patterns.

The normal and abnormal pressure wave patterns are stored in a computer system and used to real-time compare an actual injection pressure wave profile, drawn for an ongoing injection in a given tissue, cavity or organ.

The injection pressure wave profile of each injection is also stored, offering a valuable information to prove adherence to good clinical practice recommendations, for instance in case of litigation.

In the following, "during injection" means during activation of the syringe, including when the liquid exits through the needle but also when no liquid exits, due to high resistance at the needle tip. In other words, the alarm may be activated before the injection of a significant volume, at the moment when the pressure in the needle reaches a pre-set threshold.

Different way to activate the alarm or to set its threshold are provided, according to different embodiments of the present invention.

In this respect, the alarm system may be integrated in the needle, for instance in a same circuit board or chip on which the sensor is embedded and the threshold may be set in the needle. In this embodiment, the alarm may be an acoustic alarm which is emitted when the sensor detects the pressure above or below the pre-set threshold. Means for emitting the alarm may be arranged in the hub of the needle.

The threshold in the sensor may be programmable or not programmable. In case of pre-set and not programmable (rewritable) threshold, different needles may be provided with corresponding different predetermined and not changeable thresholds, each needle being adapted to a certain application (anaesthesia, etc, etc). In case of programmable needle, the sensor is associated to an interface adapted to receive a value from outside the needle, to be set as a threshold; a wireless interface device may be included in the needle, for instance embedded in the same chip with the sensor, receiving instruction and values from a programming device, to set the threshold. RFID technology may be used for this purpose.

According to different embodiments of the invention, the alarm system may be outside the needle and/or the threshold may be set and stored outside the needle.

For instance, an external device, wired or wirelessly connected to the needle, may store the threshold value and trigger the alarm, as an audio signal or video signal, when the pressure value received from the pressure sensor is above or below a predetermined value, set in the external device as a threshold, according to the application to be controlled.

Preferably, the pressure sensor is arranged at the tip portion of the needle.

According to different embodiments, the pressure sensor may be fixed inside the needle, for instance inside the thickness of the inner wall of the needle shaft or it may be fixed to an end of a wire or cable arranged inside the needle and preferably used also to transmit the pressure value. According to different embodiments, the cable may be rigid, semi rigid or flexible,

Advantageously, the external shape of the needle, its size, hub, bore, etc, may be unchanged, i.e. same shape, size, hub, bore of a standard and known needle, and just the inner part of the needle is adapted to contain the sensor. Moreover a normal syringe available on the market may be attached to the needle of the present invention, therefore allowing the operators to use its own equipment and avoiding any replacement. Furthermore, the operator may perform the injection with no changes to his normal way of injecting and with nothing complicating his gesture.

According to another embodiment of the invention, the pressure sensor is inside a tube which is arranged inside the shaft of the needle. The tube may be empty, i.e. just including the sensor and air, or filled with a liquid (with or without a membrane at his end), and the pressure measured by the sensor is the pressure exerted by the air or the liquid in the tube during injection. In such a case, the sensor does not measure the pressure of the liquid injected directly but it measure the pressure of the liquid injected indirectly through air or (another) liquid in the tube. One end of the tube may be associated to the tip of the needle and another end to the hub thereof.

The sensor may have a sensing surface adapted to increase the sensibility. For instance, the sensing surface may be perpendicular to the shaft or it may include a portion perpendicular to the shaft and/or at least a portion parallel to the bevel.

According to the present invention, the pressure sensor inside the needle is useful in different clinical scenarios and has thus many potential practical applications, in the fields of regional anesthesia, interventional pain therapy, interventional radiology and bioptic procedures. Examples can be the treatment of musculoskeletal disorders, injuries, disease, biopsies, surgical operations, etc., where injections of different kinds are performed blindly, according to anatomical landmarks or under nerve-stimulator or ultrasound guidance. These includes steroids, local anaesthetics, hyaluronic acid, alcohol, fenol, antiinflammatory drugs, normal saline (for tissues hydrodissection) or mixtures of the above. At the moment of the injection the sensor immediately detects the pressure and the eventual activation of the alarm, properly set for that specific clinical situation, informs the operator about the injection pressure generated at the needle tip, preventing him or her from inappropriately administer the drug at the wrong site causing damages related to either the drug itself (such as in case of intravascular injection) or the mechanic effect exerted by its volume in a low compliance structure (such as in case of the ischemic damage caused to nerve fibers by an intraneural local anesthetic injection during a peripheral nerve block).

According to the solution described above, the technical problem is solved by a needle including:
- a tip at one end of the needle, adapted to penetrate a body tissue;
- a hub at the other end of the needle, to connect the needle to the syringe; characterised by including:
- a pressure sensor in the needle;
- means to transmit outside the needle a pressure value sensed by the pressure sensor in the needle.

According to an embodiment of the invention, these means include a wireless interface to transmit pressure values to an external device.

According to a first embodiment of the invention, the means and the pressure sensor are embedded in the same board.

According to a second embodiment of the invention, the means and the pressure sensor are in different boards, wherein the boards are connected together, inside the needle.

According to an embodiment of the invention the means are fixed inside the needle, for instance in the thickness of an internal wall of the shaft needle, preferable in proximity of the needle or bevel.

According to another embodiment of the invention, the means includes a cable, which may be rigid or semi rigid according to different aspects of the invention, the cable comprises a first end optically or electrically connected to the pressure sensor in the needle, a second end which exit from the needle through the other end of the needle, and a portion between the first end and the second end inside the needle. Preferably, the cable is an optical fibre.

According to an embodiment of the invention, the second end of the cable includes a wireless interface to transmit wirelessly the pressure value to a device outside the needle.

Preferably, the cable is fixed to the hub at a position of the cable leaving a predetermined length of the cable inside the needle, the predetermined length substantially corresponding to a predetermined distance of the pressure sensor from the second end of the needle.

According to an embodiment of the invention, the pressure sensor is at the tip of the needle or at a predetermined distance from the tip or from a bevel of the needle, the predetermined distance from the tip or bevel being from 1% to 20% of a length of the needle.

According to another embodiment of the invention, the pressure sensor is at the second end of the needle or at a predetermined distance from the second end, the predetermined distance from the second end being from 1% to 20% of a length of the needle. In another embodiment, the pressure sensor is in the hub.

Preferably, the sensor includes a sensing surface arranged to be contacted by a fluid to be injected with the syringe, and being adapted to measure a pressure of the fluid around the bevel, and/or a variation of the fluid pressure around the bevel.

The sensing surface includes at least a portion perpendicular to an axis of the needle, the portion being towards the tip of the needle.

The technical problem mentioned above is also solved by a syringe according to the present invention, wherein the hub of the needle is integrated in or detachable-from the syringe.

The technical problem mentioned above is also solved by a system including an external device connected or connectable to the means of the needle.

According to an embodiment of the invention, the second end of the cable is connected to the external device and the pressure sensor is powered by the external device.

The external device may include a user interface to display the pressure value and further include a alarm means configured to emit an acoustic and/or audio signal when the pressure value is above or below a predetermined threshold.

Several combinations of the features indicated above with reference to one embodiment or to another embodiment of the invention may be combined. Just as an example of these combinations: the wireless interface of the sensor, which has been indicated according to one embodiment in which a wired connection with an external device is not provided, may be adopted to receive commands for storing the threshold values inside the sensor, and an external device including an alarm system to emit an audio or video system, which has been disclosed according to another embodiment, may be adopted to receive a signal from the sensor when the sensor detects a pressure above or below the threshold and to emitting the alarm outside the needle.

The needle, the syringe, the system, as well as the injection method, according to the present invention, solve many technical problems. More in details, in case of orthopedic or surgical procedures on limbs, when a nerve block is performed to anesthetize the body region, the needle gives an important information to the operator. Indeed, if the anaesthesiologist place the needle tip in a wrong position (namely intraneural or intravascular) and start the injection, the pressure sensor inside the needle immediately detects that the pressure is respectively too high or too low with respect to pre-set pressure thresholds, titrated on the normal pressure generated by tissues found in proximity of a peripheral nerve, therefore activating the alarm which alerts the operator. Moreover in case the anesthesiologist has to perform a particular nerve blocks (e.g. psoas compartment block) the injection pressure values provide a valuable information in order to achieve an optimal local anesthetic spread, since, even within a safety range, lower injection pressures prevent the local anesthetic spread from backtracking to the spinal column, resulting in unintentional epidural anesthesia.

Just in the case the pressure detected by the sensor inside the needle is not too high nor too low with respect to pre-set pressure threshold(s), the system does not alert the operator, since the position of the needle tip is considered not harmful in the given situation.

In this respect, the threshold may include an upper and/or a lower threshold and the alarm may be actuated when the upper and/or a lower threshold is detected. In all the embodiments of the invention, an upper and/or a lower threshold may be provided, depending on the application.

At the same time, the pressure sensor inside the needle may detect also that the pressure is too high and alert the operator that the needle may be in the nerve itself, to immediately stop injecting the anaesthetic fluid and avoid traumatic nerve injuries.

Advantageously, the pressure detected by the needle gives a precise indication on the position of the needle in relation to the nerve, with a high level of certainty, and guaranteeing accuracy, since the pressure is detected close to (or inside) the nerve. Therefore, significant nerve injury or nerve damage, as well as risks of systemic and local toxic complications, mechanical trauma to the nerve, ischemic injury to the nerve due the resultant increase in endoneural pressure caused by high pressures inside the nerve, and/or endoneural edema, may be avoided.

Advantageously, the needle may alert the operator when the speed of injection is too high; indeed, the sensor may detect a high pressure during injection of local anaesthetic not only as a consequence of an undesired/inappropriate location of the needle tip (for instance in a nerve) but also as a consequence of a too high speed of injection; therefore, the alarm informs the operator and the operator may immediately slow down the injection speed.

Once the speed is reduced, the alarm may stop or not, depending on the implementation of the system and desired effect.

If the alarm is not stopped also after reduction of injection speed, since the sensor still detects a high pressure, such a high pressure is probably caused by the insertion of the needle in the nerve, and therefore the injection may be immediately stopped; otherwise, if the alarm is stopped after reduction of injection speed, the operator may decide to continue with the injection, being the high pressure probably caused by an excessively high injection speed.

Depending on the situation, the alarm may be programmed to stop when the pressure returns below the threshold or it may be programmed not to stop, once the pressure has passed the threshold for the first time.

Advantageously, according to the present invention, the needle-injection line-syringe system used does not oblige the operator to change his or her habits, since it does not require a modification of the system with the insertion of an adjunctive external component. Therefore, on one hand the needle gives useful indications about the position of the needle tip, on the other hand, the operator is provided with an easy, comfortable, ergonomic tool system to perform the procedure. The alarm may be acoustic so that the operator can focus continuously on the ultrasound machine screen or the surgical field at the needle insertion point.

Finally, the pressure detected by the sensor in the needle actually corresponds to the pressure in proximity of the nerve at any given time. No means are interposed between the pressure sensor and the needle and therefore nothing interferes with and alter the pressure measurement. The needle is of course disposable and can be connected to any common injection syringe via a standard connection, like any other needle.

According to a further aspect of the invention, a plurality of pressure values measured by the sensor in the needle are stored, preferably in association with corresponding injection times. Storage is made on a memory on board with the sensor or in a memory of the external device, according to different embodiment of the invention. The pressure values may be used as input for a processor or software application adapted to create pressure profiles, pressure monitoring or laboratories tests.

A plurality of profiles, each comprising a plurality of pressure values associated to a respective injection, may be stored and provided for analysis, for instance to identify the best profile of injection for each kind of injection among a plurality of injections made in the past. According to the present invention, the profile of a single injection may be displayed in real time during injection, for instance on a same display wherein an echography is displayed. The operator may monitor the echography as well as the pressure profile to control that he is performing properly the injection. In one embodiment, the ideal pressure profile may be displayed near the real time profile, so that the operator may try to reproduce the ideal profile, while injecting.

In one embodiment, the external device may be a smartphone, a portable device, an iPhone an iPad, or similar and the software application may be installed and executed on board the external device.

Finally, the system includes a memory card to store the injection pressure wave profile generated throughout the whole procedures. This information can be kept in the patient dossier together with the description of the procedure by the clinician. This can constitute a proof of good clinical practice in case of eventual litigation following side-effects or complications after an injection.

Further features and advantages of the needle, the syringe and the system according to the present invention are described with reference to the annexed drawings, just given for exemplification and without limiting the scope of protection of the present invention.

### Brief description of the drawings:

Figure 1 schematically represents a needle according to the prior art.
Figure 2 schematically represents a needle according to the present invention.
Figure 3 is an enlarged cross section of portion A of the needle of figure 2.
Figure 4 is the portion of the needle of figure A, according to a first embodiment of the present invention.

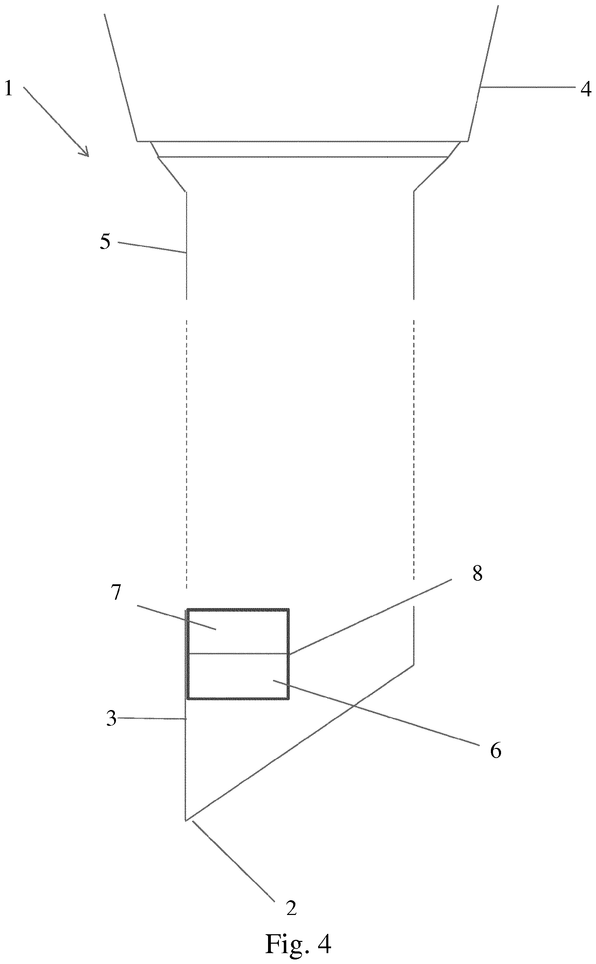

Figure 4 is the portion of the needle of figure A, according to a second embodiment of the present invention.

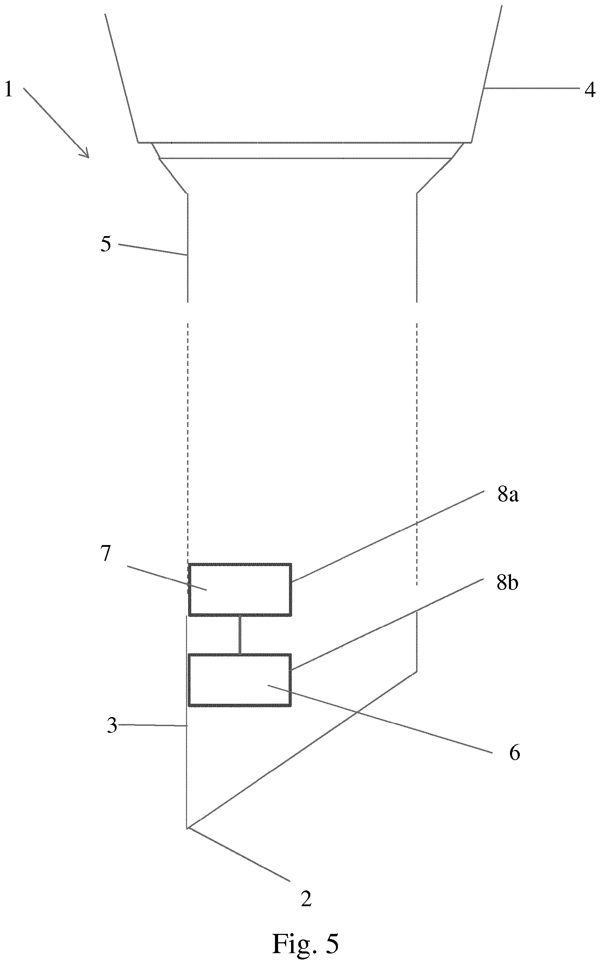

Figure 5 is the portion of the needle of figure A, according to a third embodiment of the present invention.

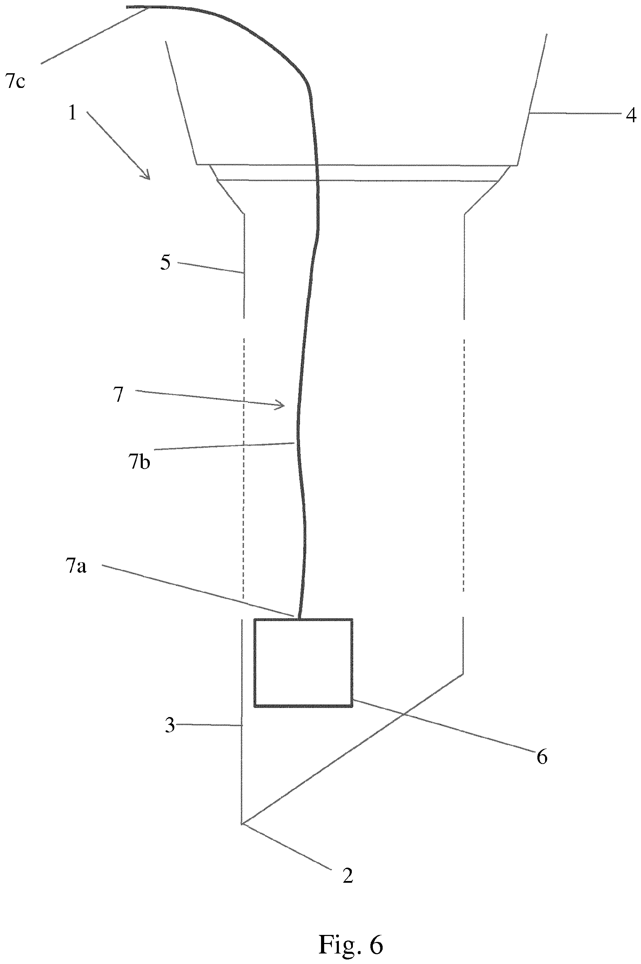

Figure 6 is the portion of the needle of figure A, according to a fourth embodiment of the present invention.

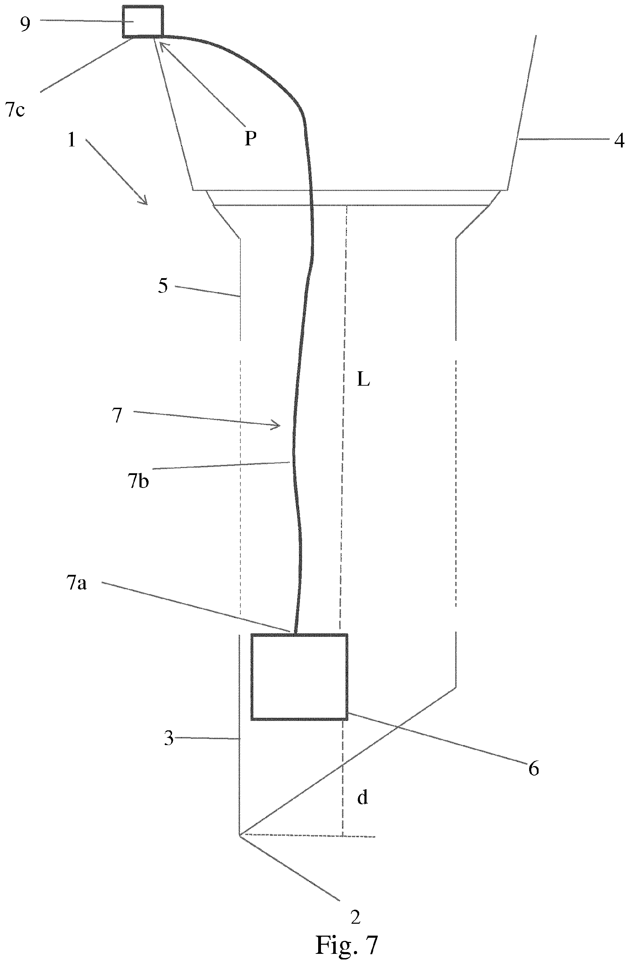

Figure 7 is the portion of the needle of figure A, according to a fifth embodiment of the present invention.

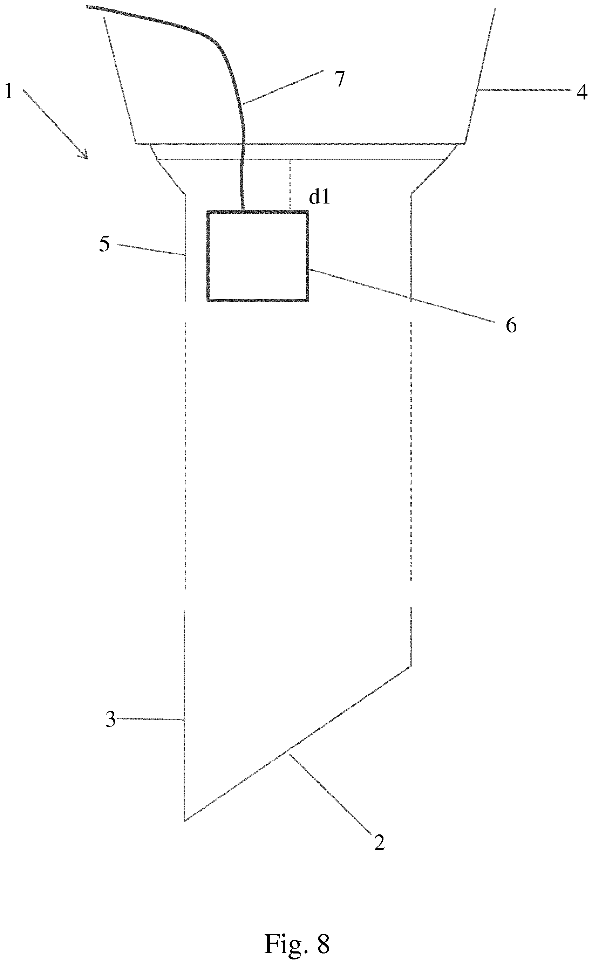

Figure 8 is the portion of the needle of figure A, according to a sixth embodiment of the present invention.

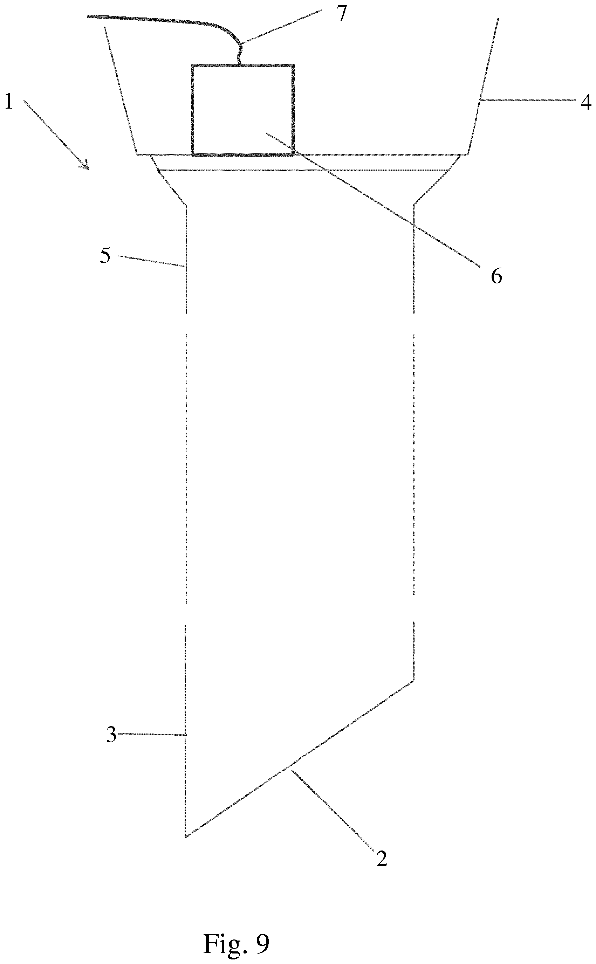

Figure 9 id the portion of the needle of figure A, according to a seventh embodiment of the present invention.

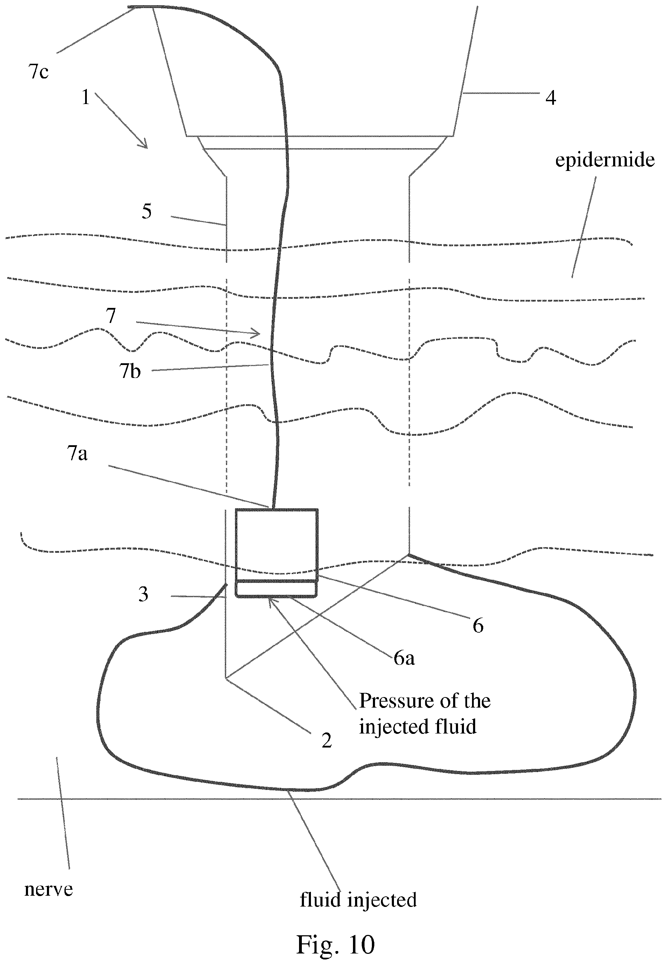

Figure 10 is the portion of the needle of figure A, during use.

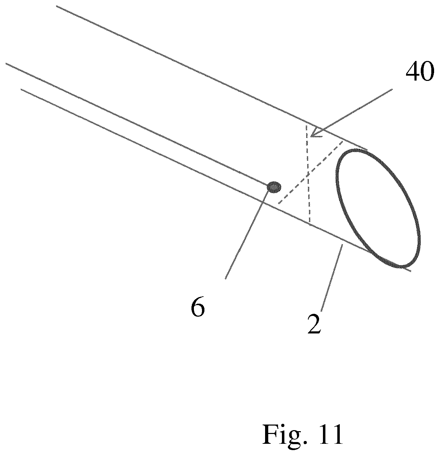

Figure 11 is the needle of figure A, according to an embodiment of the present invention.

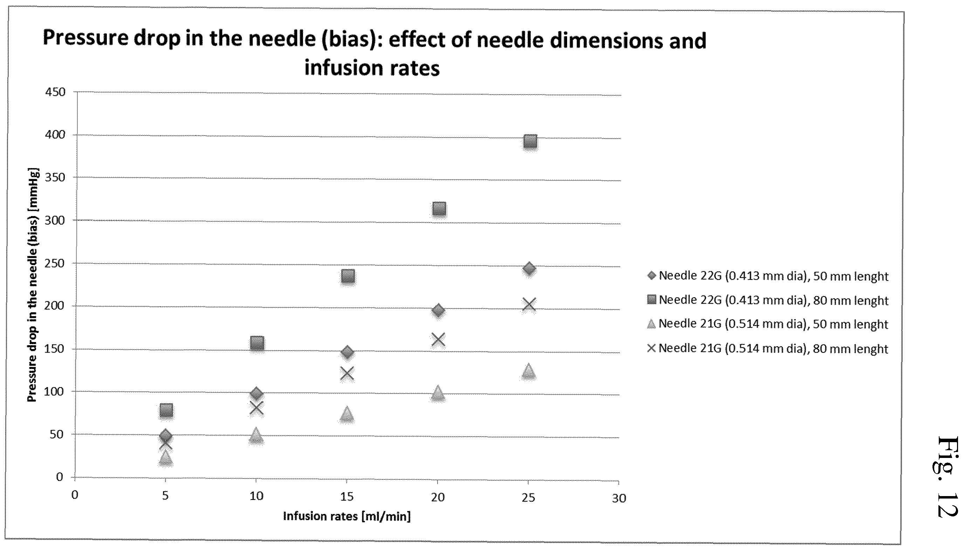

Figure 12 is a diagram representing the pressure drop in the needle as a function of the infusion rates and for different needle dimensions (internal diameter and length).

### Detailed description:

With reference to the annexed drawings, it is hereafter disclosed and indicated with reference number 1 a needle according to the present invention, and more particularly a needle adapted to be plugged to a tube and/or to a syringe including a liquid, the liquid being directed to be injected in the body of a patient for a predetermined application. Just as example and without limiting the scope of protection of the invention, the application may be an anaesthesia and the liquid may be a local anaesthetic; in such application, it is important to avoid that the liquid is not injected in a nerve.

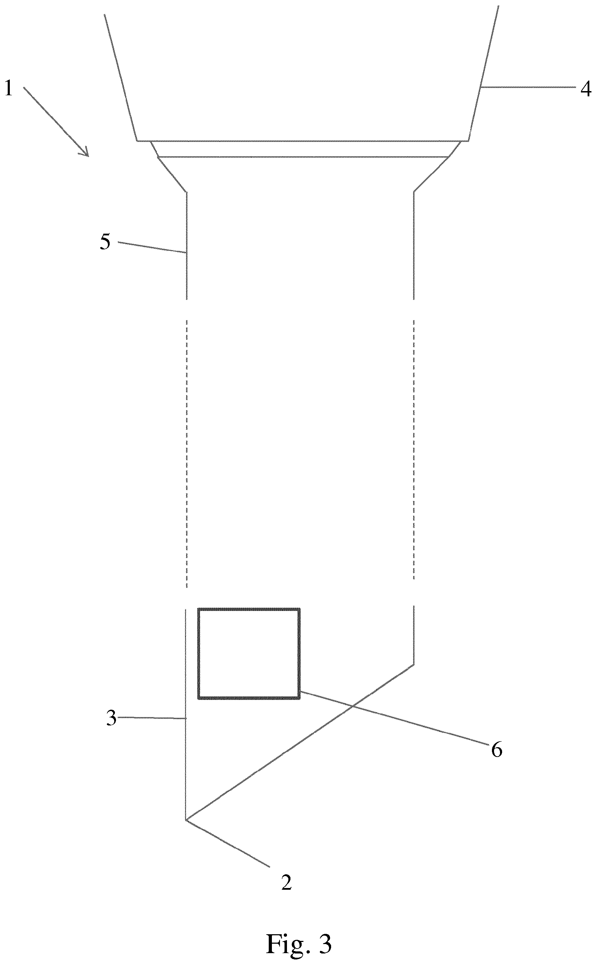

According to the present invention, the needle 1 has a tip 2 at one end 3, a hub 4 at the other end 5, as schematically represented in fig. 2. Fig 3, is an enlarged cross section of a portions A of fig. 1, representing a sensor 6 inside the needle. The sensor is in the needle shaft. Preferably, the sensor is at the tip portion 2 of the needle.

During use, the needle passage through body tissues, as represented in fig. 10; in this figure the needle has passed through the epidermises and the tip 2 of the needle is inside a nerve, where the anaesthetic liquid should not be injected. The nerve makes resistance to the liquid injected which is greater than a resistance exerted on the liquid from other tissues or body fluids, for instance the pressure exerted by a muscle.

According to the invention, when the syringe is actuated, i.e. when the plunger is pressed, the liquid pass from the liquid container in the syringe into the needle shaft, wherein the pressure sensor detect the pressure of the liquid. The pressure value detected by the sensor is compared to a threshold value which is associated to the kind of injection, i.e. to the kind of application. For instance, the threshold for the application "anaesthesia" is set to a value X which is less than the pressure exerted by a nerve into which it is to be avoided the injection of the anaesthetic liquid.

With reference to figure 3, the threshold is stored inside the pressure sensor 6, the same being also provided with an alarm system, adapted to emit an acoustic signal, when the threshold is reached. The sensor is fixed inside the needle shaft, for instance in a thickness of the needle (not represented). The threshold may be pre-set and not changeable or it may be re-written in a read-write memory of the sensor.

According to the present invention, means 7 adapted to transmit outside the needle a pressure value sensed by the pressure sensor 6 in the needle.

Fig. 4 schematically represents an embodiment of the means 7, wherein the means 7 includes a wireless interface to transmit wirelessly the pressure value to a device outside the needle (not represented). More particularly, the means 7 and the pressure sensor 6 may be embedded on a same board 8. The board 8 may be fixed inside the needle, for instance in the thickness of an internal wall of the needle shaft or on the internal wall.

With reference to fig. 5 and to another embodiment of the invention, the means 7 and the pressure sensor 6 are on separate boards 8a, 8b, and the separate boards are connected together, inside the needle, for instance through a wired connection. The separate boards 8a, 8b are also connected to the needle, for instance on the internal wall or in the thickness thereof.

According to a preferred embodiment of the invention, the sensor 6 is connected by wire, (rigid or semi rigid according to different aspects of the invention) to an external device, as represented in fig. 6. In this case, the means 7 includes a cable, the cable comprising a first end 7a optically or electrically connected to the pressure sensor 6 in the needle, a second end 7c which exit from the needle through the other end 5 of the needle, and a portion 7b between the first end 7a and the second end 7c inside the needle. The cable may be an optical fibre.

Nothing prevents that the second end of the cable 7c includes a wireless interface 9 (fig. 7) to transmit wirelessly the pressure value to a device outside the needle, instead of being wired connected to the external device. This solution is advantageous because the wireless interface is outside the needle, i.e. does not reduce the space in the free needle, and does not required a wired connection to external device, which is preferred for free movements of the user during injection.

The cable may be fixed to the hub 4 at a position P of the cable leaving a predetermined length L of the cable inside the needle, predetermined length substantially corresponding to a predetermined distance of the pressure sensor 6 from the second end 5 of the needle, for instance as represented in figure 7. The same apply to the embodiment of figure 6. In these embodiments, the sensor is at a predetermined distance d from the tip or from the bevel of the needle. Preferably, the predetermined distance from the tip or bevel is from 1% to 20% of a length of the needle.

As specified above, the rigidity of the cable is chosen depending on the embodiment of the needle, for instance, for the embodiment schematically represented in figure 6, wherein the sensor is maintained in a floating position inside the needle shaft by the cable, it is appropriate that the cable is rigid, so as to avoid fluctuation of the sensor during liquid injections, and corresponding pressures variations and errors in said detections. In fig. 6, the cable is represented as partially curved but nothing prevents that it is at the end of a riding and rectilinear cable.

With reference to figure 8 and to another embodiment, the pressure sensor 6 is at the second end 5 of the needle or at a predetermined distance from the second end 5, the predetermined distance from the second end 5 being from 1% to 20% of a length of the needle. In one embodiment the pressure sensor 6 is in the hub, as schematically represented in fig. 9. Such embodiments are preferred when high pressures are involved, since in such applications, the sensor 6 may easily detect the pressure also at a distance from the tip substantially corresponding to the length of the shaft but at the same time it is more protected inside the needle, for instance from contacts with body tissues, since more retracted in the needle and distant from the bevel.

In a preferred embodiment of the invention, the cable is an optical fibre connected at one end with the sensor and at another end with an external device, and the sensor is powered by the external device. The external device may be a personal computer or a smartphone or a portable device including means to set and store a threshold in a memory, means to receive a pressure value through the optical cable from the sensor. Said means of the external device further include a comparator to compare the threshold with the pressure value and an alarm system, to alert the user when the pressure value is above or below the threshold, depending on the application.

In figure 11, another embodiment of the present invention is schematically represented, wherein means 40 for protecting the sensor are provided within the needle, for instance a protection grid 40 or a mesh 40 inside the shaft, between the tip 2 and the sensor 6. The protections grid 40 protects the sensor inside the needle, especially during the insertion of the needle in the body tissues, when tissues may enter the needle.

Figure 12 is a diagram schematically representing the pressure drop in the needle as a function of the infusion rate, in connection with four different needles distinguished from their diameter and lengths. Advantageously, according to the present invention, the pressure measured by the sensor at the tip of the needle is the real pressure of the liquid at the tip, which is substantially equal to the pressure of the nearby body portion in which the liquid is injected, and therefore gives precise information on such body portion, being any body portion characterized by a corresponding pressure.

To the contrary, according to the device of the prior art, the pressure measures in a casing arranged between the syringe and the needle is different from the real pressure at the tip, due to pressure drops which, among other factors, depends on the needle size and injection rates, as schematically represented in figure 12.

Still according to the present invention, a method to control the injection of a liquid through a needle is disclosed. The method comprises the steps of penetrating a body tissue with a needle having a tip 2 at one of its end and a hub 4 at the other end 5, the hub attaching the needle to a tube or to a directly syringe used to execute the injection. Once the needle has been positioned into the anatomical region wherein the injection should be made, the operator begins to inject the drug through the syringe.

A pressure sensor 6 in the needle, preferably in the tip 2 of the needle, measures the pressure of the liquid in the needle and means 7 transmit the pressure value outside the needle. The pressure value is compared with a pressure threshold, which is previously set, based on the type of injection which is performed, and therefore on the base of the anatomical region wherein the injection should be made. If the pressure value is not compatible with the pressure threshold, an alarm is emitted and the operator may immediately stop injecting.

For instance, if the type of the injection is in the setting of regional anesthesia, the liquid (i.e. the local anesthetic) should be injected near the nerve to be blocked but not in the nerve itself. Therefore, the pressure threshold is the typical pressure near the nerve, which is lower than the typical pressure inside the nerve. During injection, if the sensor detects at the tip of the needle a pressure greater than the pressure threshold, the alarm is emitted. Moreover, in order to avoid the backtrack of local anesthetic to the epidural space (in case of injection close to the vertebral column), the exceeding of a pre-set pressure threshold also lead to an alarm emission by the system.

Advantageously, the system of the present invention, further than a controlling pressure threshold adapted for the injections, is also adapted to collect pressure profiles associated to the injections. More particularly, the external device connected to the sensor in the needle stores a plurality of pressure values detected by the sensor during an injection at corresponding injection times, and includes means to build a profile of pressures associated to the injection, the profile being representable as a curve on a display. The means to build the profile of pressures includes for instance a software application for PC or an application for apps and the display may be a monitor of a medical device, a pc or a portable device, such as an iPhone, iPad or tablet.

The external device stores a plurality of profiles of pressures associated to a corresponding plurality of injections. Injections made by a same operator may be grouped.

The external device, for instance the above mentioned software application, may include a user interface adapted to associate a user's score to a profile of pressures, the score being dependent on the result of the injection made by the user. For example, an anaesthesiologist, based on its feeling or the reaction of the patient or his personal feed-back or medical examinations, may type the user interface and set the score. Preferably in the same user interface and in association to the injection performed, the user sets a field associated to the kind of injection, For instance, the anaesthesiologist may set the field to anaesthesia for all his injection; in one embodiment, the field is automatically set for the user, based on the kind of injections he makes.

In the particular case of peripheral nerve blocks, the fine, accurate and precise real-time measurement of the injection pressures at the needle tip can help reshaping the practice of regional anesthesia. A combination of information related to the motor response to nerve stimulation and the injection pressure can not only help the operator to avoid complication related to a intraneural injection but also help him to optimize the needle tip positioning, in order to maximizing efficacy of the nerve block while preserving safety, through a sub-perineural but extra-epineural injection of local anesthetic. Perineurium is the sheath encapsulating the nerve to protect it and constitute a natural barrier to the spread of the local anesthetic, thus slowing its onset and potentially jeopardizing its clinical outcome. Epineurium instead is the relatively anelastic inner sheath which delimitates the nerve and shell not be penetrated by the needle tip in order to avoid increase of intraneural pressure following the injection of local anesthetic, potentially leading to an ischemic damage due to the collapse of vasa-nervorum. The ideal spot to inject the local anesthetic would be the space between the perineurium and the epineurium: this would optimize the clinical effect of the block, trespassing the barrier to the local anesthetic spread represented by the perineurium, minimizing the necessary total volume and significantly reducing the onset time.

Ultrasound guided regional anesthesia has become a widely accepted standard in contemporary clinical practice. Real-time ultrasound guidance consent to visualize the needle and the target structure, visualizing eventual obstacles on the path (such as vessels, the pleura, organs, etc.). However echography is operator-dependant and the resulting image quality is heavily affected by many variables affecting the acoustic windows (such as structures depth, the angle between the needle and the ultrasound bean axe, anisotropy of nerves). The needle tip position is thus often difficult to visualize with a reasonable degree of confidence and this translates into many reports of accidental intraneural injections and nerve lesions under ultrasound guidance.

Moreover the definition actually provided by ultrasound machines is not sufficient to discriminate millimetric spaces and structures, such as the perineural and epineural sheaths and the space in between them.

With these premises, the electrophysiologic information provided by the nerve-stimulator, together with a precise information about the injection pressure at the needle tip, are usefully complementing the ultrasound image to provide a broader picture. A minimal electric threshold to elicit electric response (<0.2 mA) is helpful to exclude a subepineural placement of the needle tip; likely an appropriate injection pressure at the needle tip confirm a correct needle tip position. In this framework, the injection pressure at the needle tip appears not only as a safety standard, but also as a useful tool to help in optimizing needle tip positioning in the ideal spot to maximize efficacy while preserving safety.

Patients' safety must always be the first concern for any medical professional. The introduction of ultrasound guidance alone, in clinical fields using interventional procedures such as regional anesthesia, has not been shown as effective per se in reducing the most feared complications, such as persistent and permanent nerve injuries. Continuous injection pressure precise measurement at the needle tip is a valuable tool to increase safety during invasive procedures, giving precious information about the location of the needle tip with respect to adjacent structures.

The existent device in the market does not provide this information:
- it does not measure the injection pressure at the needle tip, but along the injection line, which is less precise being affected by many different confusing variables;
- it does not measure the injection pressure continuously nor accurately, but it just provides three threshold which few studies have associated to different level of risk, regarding solely an intraneural positioning of the needle tip (thus it cannot be used to detect intravascular injections or to assist the operator in an optimal needle tip positioning);
- it does not provide any information about an injection pressure lower than normal;
- it is not ergonomically friendly, obliging the operator to intermittently check the visual indicator, which implies a momentary blind spot in ultrasound guidance with consequent danger of needle tip accidental displacement.

The new system described here has been specifically conceived to address and solve all these problems, with the double aim to increase both safety standard and accuracy of interventional procedure.

## Claims

1. Needle (1) for a syringe, including:
- a tip (2) at one end (3) of the needle, adapted to penetrate a body tissue during usage of the syringe;
- a hub (4) at the other end (5) of the needle, to attach the needle to a tube or to the syringe;
including:
- a pressure sensor (6) in the needle;
- means (7) to transmit outside the needle a pressure value sensed by the pressure sensor (6) in the needle, **characterized by** the fact that
said means (7) are fixed inside the hub (4)
and include a cable, the cable comprising a first end (7a) optically or electrically connected to the pressure sensor (6) in the needle, a second end (7c) which exit from the needle through said other end (5) of the needle, and a portion (7b) between the first end (7a) and the second end (7c) inside the needle,
wherein the pressure sensor is not fixed to the shaft of the needle and wherein the first end (7a) of the cable is attached to the pressure sensor (6) and the cable avoid fluctuation of the pressure sensor (6) in the needle.

2. Needle according to claim 1 wherein the pressure sensor (6) is at the tip (2) of the needle or at a predetermined distance (d) from the tip or from a bevel of the needle, said predetermined distance from the tip or bevel being from 1% to 20% of a length of the needle.

3. Needle according to claim 1 wherein the pressure sensor (6) is at the second end (5) of the needle or at a predetermined distance from the second end (5), said predetermined distance from the second end (5) being from 1% to 20% of a length of the needle.

4. Needle according to claims 1 to 3 wherein the sensor includes a sensing surface (6a) arranged to be contacted by a fluid to be injected with the syringe, and being adapted to measure a pressure of the fluid around the bevel, and/or a variation of the fluid pressure around the bevel.

5. Needle according to claim 4 wherein the sensing surface includes at least a portion perpendicular to an axis of the needle, said portion being towards the tip of the needle.

6. Needle according to claim 1, including means (40) for protecting the sensor in the needle, the protection means (40) being arranged between the tip (2) of the needle and the sensor (6).

7. Needle according to claim 1, wherein the cable is fixed to the hub (4) at a position (P) of the cable leaving a predetermined length (L) of the cable inside the needle, said predetermined length substantially corresponding to a predetermined distance of the pressure sensor (6) from the second end (5) of the needle.

8. Needle according to claim 7, wherein the second end of the cable (7c) includes a wireless interface (9) to transmit wirelessly the pressure value to a device outside the needle.

9. Needle according to claim 1, wherein the cable is an optical fibre.

10. Needle according to claim 9, wherein the pressure sensor (6) is a Fabry-Perot optical fibre sensor.

11. System including an device connected or connectable to the means (7) of the needle according to any one of claims 1 to 10, to control the pressure in the needle.

12. System of claim 11, wherein said second end of the cable is connected to the device and the pressure sensor is powered by the device.

13. System according to claims 11 or 12, wherein the device includes an alarm system configured to emit an acoustic and/or audio and/or video signal when the pressure value is above or below a predetermined threshold.

14. System according to any one of claims 11 to 13, wherein the device includes a user interface to display the pressure values and/or pressure curve in real-time.

15. System according to any one of claims 11 to 14, wherein the device includes a wireless interface adapted to be connected to a portable device or to a PC to transmit at least said pressure values.

## Patentansprüche

1. Nadel (1) für eine Spritze, enthaltend:
- eine Spitze (2) an einem Ende (3) der Nadel, die geeignet ist, während der Benutzung der Spritze in ein Körpergewebe einzudringen,
- eine Nabe (4) am anderen Ende (5) der Nadel, um die Nadel an einem Rohr oder an der Spritze zu befestigen,
enthaltend:
- einen Drucksensor (6) in der Nadel,
- Mittel (7) zum Übertragen eines von dem Drucksensor (6) in der Nadel erfassten Druckwerts nach außen, **dadurch gekennzeichnet, dass**
die Mittel (7) im Inneren der Nabe (4) befestigt sind
und ein Kabel enthaltend, wobei das Kabel ein erstes Ende (7a) umfasst, das optisch oder elektrisch mit dem Drucksensor (6) in der Nadel verbunden ist, ein zweites Ende (7c), das aus der Nadel durch das andere Ende (5) der Nadel austritt, und einen Abschnitt (7b) zwischen dem ersten Ende (7a) und dem zweiten Ende (7c) innerhalb der Nadel,
wobei der Drucksensor nicht am Schaft der Nadel befestigt ist und
wobei das erste Ende (7a) des Kabels am Drucksensor (6) befestigt ist und das Kabel Schwankungen des Drucksensors (6) in der Nadel vermeidet.

2. Nadel nach Anspruch 1, wobei sich der Drucksensor (6) an der Spitze (2) der Nadel oder in einem vorbestimmten Abstand (d) von der Spitze oder von einer Abschrägung der Nadel befindet, wobei der vorbestimmte Abstand von der Spitze oder Abschrägung 1 % bis 20 % der Länge der Nadel beträgt.

3. Nadel nach Anspruch 1, wobei sich der Drucksensor (6) am zweiten Ende (5) der Nadel oder in einem vorbestimmten Abstand vom zweiten Ende (5) befindet, wobei der vorbestimmte Abstand vom zweiten Ende (5) zwischen 1% und 20% der Länge der Nadel beträgt.

4. Nadel nach den Ansprüchen 1 bis 3, wobei der Sensor eine Abtastfläche (6a) enthält, die so angeordnet ist, dass sie mit einem mit der Spritze zu injizierenden Fluid in Kontakt kommt, und die dazu geeignet ist, einen Druck des Fluids um die Abschrägung herum und/oder eine Veränderung des Fluiddrucks um die Abschrägung herum zu messen.

5. Nadel nach Anspruch 4, wobei die Abtastfläche mindestens einen Teil senkrecht zu einer Achse der Nadel enthält, wobei dieser Teil zur Nadelspitze hin gerichtet ist.

6. Nadel nach Anspruch 1, enthaltend Mittel (40) zum Schutz des Sensors in der Nadel, wobei die Schutzmittel (40) zwischen der Spitze (2) der Nadel und dem Sensor (6) angeordnet sind.

7. Nadel nach Anspruch 1, wobei das Kabel an der Nabe (4) an einer Position (P) des Kabels befestigt ist, die eine vorbestimmte Länge (L) des Kabels innerhalb der Nadel belässt, wobei die vorbestimmte Länge im wesentlichen einem vorbestimmten Abstand des Drucksensors (6) vom zweiten Ende (5) der Nadel entspricht.

8. Nadel nach Anspruch 7, wobei das zweite Ende des Kabels (7c) eine drahtlose Schnittstelle (9) enthält, um den Druckwert drahtlos an ein Gerät außerhalb der Nadel zu übertragen.

9. Nadel nach Anspruch 1, wobei das Kabel eine optische Faser ist.

10. Nadel nach Anspruch 9, wobei der Drucksensor (6) ein Fabry-Perot-Lichtleitfaser-Sensor ist.

11. System enthaltend eine Vorrichtung, die mit den Mitteln (7) der Nadel nach einem der Ansprüche 1 bis 10 verbunden oder verbindbar ist, um den Druck in der Nadel zu kontrollieren.

12. System nach Anspruch 11, wobei das zweite Ende des Kabels mit dem Gerät verbunden ist und der Drucksensor von dem Gerät mit Strom versorgt wird.

13. System nach Anspruch 11 oder 12, wobei die Vorrichtung ein Alarmsystem aufweist, das so konfiguriert ist, dass es ein akustisches und/oder Audio- und/oder Videosignal aussendet, wenn der Druckwert über oder unter einem vorbestimmten Schwellenwert liegt.

14. System nach einem der Ansprüche 11 bis 13, wobei das Gerät eine Benutzerschnittstelle zur Anzeige der Druckwerte und/oder der Druckkurve in Echtzeit enthält.

15. System nach einem der Ansprüche 11 bis 14, wobei das Gerät eine drahtlose Schnittstelle enthält, die an ein tragbares Gerät oder an einen PC angeschlossen werden kann, um mindestens die Druckwerte zu übermitteln.

## Revendications

1. Aiguille (1) pour seringue, incluant :
- une pointe (2) au niveau d'une extrémité de l'aiguille, adaptée à pénétrer un tissu du corps pendant l'utilisation de la seringue,
- un raccord (4) au niveau de l'autre extrémité (5) de l'aiguille, destiné à fixer l'aiguille à un tube ou à la seringue,
incluant :
- un détecteur de pression (6) dans l'aiguille,
- un moyen (7) de transmettre une valeur de pression détectée par le détecteur de pression (6) dans l'aiguille, à l'extérieur de l'aiguille, **caractérisé en ce que**
le dit moyen (7) est fixé à l'intérieur du raccord (4)
et inclut un câble, le câble comprenant une première extrémité (7a) connectée de manière optique ou électrique au détecteur de pression (6) dans l'aiguille, une deuxième extrémité (7c) qui sort de l'aiguille par la dite autre extrémité (5) de l'aiguille, et une partie (7b) entre la première extrémité (7a) et la deuxième extrémité (7c) à l'intérieur de l'aiguille,
où le détecteur de pression n'est pas fixé à l'axe de l'aiguille et où la première extrémité (7a) du câble est fixée au détecteur de pression (6) et le câble évite le déplacement du détecteur de pression (6) dans l'aiguille.

2. Aiguille selon la revendication 1, **caractérisée en ce que** le détecteur de pression (6) est au niveau de la pointe (2) de l'aiguille ou à une distance prédéterminée (d) de la pointe ou d'un biseau de l'aiguille, la dite distance prédéterminée avec la pointe ou le biseau étant comprise entre 1% et 20% de la longueur de l'aiguille.

3. Aiguille selon la revendication 1, **caractérisée en ce que** le détecteur de pression (6) est au niveau de la deuxième extrémité (5) de l'aiguille ou au niveau d'une distance prédéterminée avec la deuxième extrémité (5), la dite distance prédéterminée avec la deuxième extrémité (5) étant comprise entre 1% et 20% de la longueur de l'aiguille.

4. Aiguille selon les revendications 1 à 3, **caractérisée en ce que** le détecteur inclut une surface de détection (6a) disposée pour être mise en contact avec un fluide qui doit être injecté à l'aide de la seringue et étant adaptée à mesurer la pression du fluide autour du biseau et/ou une variation de la pression du fluide autour du biseau.

5. Aiguille selon la revendication 4, **caractérisée en ce que** la surface de détection inclut au moins une partie perpendiculaire à un axe de l'aiguille, la dite partie étant en direction de la pointe de l'aiguille.

6. Aiguille selon la revendication 1, incluant un moyen (40) de protection du détecteur dans l'aiguille, le moyen de protection (40) étant disposé entre la pointe (2) de l'aiguille et le détecteur (6).

7. Aiguille selon la revendication 1, **caractérisée en ce que** le câble est fixé au raccord (4) au niveau d'une position (P) du câble laissant une longueur prédéterminée (L) du câble à l'intérieur de l'aiguille, la dite longueur prédéterminée correspondant essentiellement à une distance prédéterminée entre le détecteur de pression (6) et la deuxième extrémité (5) de l'aiguille.

8. Aiguille selon la revendication 7, **caractérisée en ce que** la deuxième extrémité du câble (7c) inclut une interface sans fil (9) pour transmettre sans fil la valeur de la pression à un dispositif à l'extérieur de l'aiguille.

9. Aiguille selon la revendication 1, **caractérisée en ce que** le câble est une fibre optique.

10. Aiguille selon la revendication 9, **caractérisée en ce que** le détecteur de pression (6) est un détecteur à fibre optique Fabry-Pérot.

11. Système incluant un dispositif connecté ou connectable au moyen (7) de l'aiguille selon l'une quelconque des revendications 1 à 10, pour contrôler la pression dans l'aiguille.

12. Système selon la revendication 11, **caractérisé en ce que** la dite deuxième extrémité du câble est connectée au dispositif et le détecteur de pression est alimenté en énergie par le dispositif.

13. Système selon les revendications 11 ou 12, **caractérisé en ce que** le dispositif inclut un système d'alarme configuré pour émettre un signal acoustique et/ou audio et/ou vidéo quand la valeur de la pression est supérieure ou inférieure à un seuil prédéterminé.

14. Système selon l'une quelconque des revendications 11 à 13, **caractérisé en ce que** le dispositif inclut une interface utilisateur pour afficher les valeurs de pression et/ou la courbe de pression en temps réel.

15. Système selon l'une quelconque des revendications 11 à 14, **caractérisé en ce que** le dispositif inclut une interface sans fil adaptée à être connectée à un dispositif portable ou à un PC pour transmettre au moins les dites valeurs de la pression.
